# EUROPÄISCHE PATENTANMELDUNG

(11) **EP 4 659 782 A1**
(43) Veröffentlichungstag der Anmeldung: **10.12.2025**
(21) Anmeldenummer: 25180343.3
(22) Anmeldetag: 03.06.2025
(51) Int. Cl.: A61M 15/00, A61M 15/08

(54) **INHALATIONSVORRICHTUNG**

(30) Priorität: 05.06.2024 AT 504552024
(71) Anmelder: Skyline Handels GmbH, 1070 Wien (AT)
(72) Erfinder: Peninger, Eduard, Wien (AT)
(74) Vertreter: Puchberger & Partner Patentanwälte

(57) **Zusammenfassung**

Die Erfindung betrifft eine Inhalationsvorrichtung (1) umfassend einen Kupplungsabschnitt (2), einen Förderabschnitt (3) und einen Ansetzabschnitt (4), wobei der Kupplungsabschnitt (2) eine Einlassöffnung (5) aufweist und zur lösbaren Befestigung der Inhalationsvorrichtung (1) an einer Öffnung eines Behälters eingerichtet ist, wobei der Ansetzabschnitt (4) zum Ansetzen an ein Nasenloch eingerichtet ist und eine Ausgabeöffnung (6) aufweist, wobei zwischen Einlassöffnung (5) und Ausgabeöffnung (6) ein durchgängiger Förderabschnitt (3) verläuft, wobei sich zumindest ein Luftzufuhrkanal (7) von einer Luftzufuhröffnung (8) an der Außenseite der Inhalationsvorrichtung in den Förderabschnitt (3) erstreckt, wobei der Förderabschnitt (3) einen gekrümmten Förderkanal (9) umfasst, wobei zwischen Kupplungsabschnitt (2) und Förderkanal (9) ein Verwirbelungsabschnitt (10) vorgesehen ist, in den der Luftzufuhrkanal (8) mündet, und wobei im Verwirbelungsabschnitt (10) eine Verwirbelungsvorrichtung (14) vorgesehen ist, die Durchlassöffnungen (15) aufweist, die einen Gasdurchtritt erlauben. Ferner betrifft die Erfindung ein System umfassend einen Behälter, an dem eine erfindungsgemäße Inhalationsvorrichtung (1) befestigt ist.

## Beschreibung

Die vorliegende Erfindung betrifft eine Inhalationsvorrichtung sowie ein System, das eine Inhalationsvorrichtung und einen Behälter umfasst oder daraus besteht.

Inhalationsvorrichtungen werden im Stand der Technik zur Inhalation von Substanzen, wie Gasen und/oder Aerosolen eingesetzt, die in der Regel einen Wirkstoff enthalten, der dem Körper über die Atemwege zugeführt werden soll. Bei dem Wirkstoff kann es sich um einen Arzneistoff handeln, der eine therapeutische Wirkung aufweist. Der Wirkstoff kann auch ein anderweitiger Riechstoff sein, der eine bestimmte Wirkung auf den menschlichen Körper ausübt.

Dazu werden Inhalationsvorrichtungen typischerweise an Behältern angebracht, die den Wirkstoff in teilweise gasförmigem bzw. aerosolförmigem Zustand enthalten. Der Wirkstoff kann im Behälter zusätzlich in einem weiteren Aggregatzustand vorliegen. Beispielsweise enthält der Behälter den Wirkstoff in flüssigem oder festem Zustand, der bei Normbedingungen teilweise in den gasförmigen Zustand übergeht.

Der Behälter enthält den Wirkstoff typischerweise in einer für mehrere Dosen ausreichender Menge. Zur Lagerung wird der Behälter mit einem Verschluss, der an seiner Öffnung angebracht wird, verschlossen. Soll eine Dosis abgegeben werden, so wird der Verschluss von der Öffnung abgenommen und an seiner Stelle die Inhalationsvorrichtung angebracht. Sodann wird die Inhalationsvorrichtung mit einer Ausgabeöffnung an der Nase angesetzt und die Person atmet durch die Inhalationsvorrichtung hindurch die Substanz ein.

Derartige Inhalationsvorrichtungen zeichnen sich unter anderem durch ihren einfachen Aufbau und ihre simple Verwendbarkeit aus.

Um das Einatmen zu vereinfachen ist es bekannt Öffnungen vorzusehen, durch die Luft von außerhalb des Behälters in die Inhalationsvorrichtung eingesaugt werden kann, wenn durch das Einatmen ein Unterdruck an der Ausgabeöffnung der Inhalationsvorrichtung anliegt. Die so zugeführte Luft wird in den Behälter geführt und bei fortgesetztem Einatmen weiter durch die Inhalationsvorrichtung zur Ausgabeöffnung befördert. Mit diesem Luftstrom wird die Beförderung des Wirkstoffs vom Inneren des Behälters in Richtung Ausgabeöffnung verbessert.

Bei starkem Einatmen kann es in bekannten Inhalationsvorrichtungen des Standes der Technik jedoch vorkommen, dass der eingesaugte Luftstrom die im Behälter befindliche Flüssigkeit aufwirbelt und Flüssigkeitstropfen erzeugt, die dann inhaliert werden können. Dies kann die Dosierung des Wirkstoffs erheblich beeinflussen, zumindest ist es jedoch für die Person unangenehm Flüssigkeit einzuatmen und kann abhängig vom Wirkstoff auch mit Gesundheitsrisiken verbunden sein.

Als eine Aufgabe der Erfindung kann es daher erachtet werden die Nachteile des Stands der Technik zumindest teilweise zu überwinden und insbesondere eine Inhalationsvorrichtung zu schaffen, die eine erhöhte Verwendungssicherheit aufweist.

Diese und weitere Aufgaben werden gegebenenfalls durch Bereitstellen einer Inhalationsvorrichtung mit einem gekrümmten Förderkanal gelöst. Insbesondere kann vorgesehen sein, dass der Förderkanal helixförmig verläuft.

Erfindungsgemäß kann die Inhalationsvorrichtung einen Kupplungsabschnitt, einen Förderabschnitt und einen Ansetzabschnitt aufweisen, wobei der Kupplungsabschnitt an einem Ende und der Ansetzabschnitt an dem anderen Ende der Inhalationsvorrichtung vorgesehen sein kann.

Der Förderabschnitt kann zwischen den beiden Enden der Inhalationsvorrichtung, insbesondere zwischen dem Kupplungsabschnitt und dem Ansetzabschnitt angeordnet sein und er kann den Kupplungsabschnitt und den Ansetzabschnitt fluidleitend verbinden.

Der Kupplungsabschnitt weist bevorzugt eine Einlassöffnung auf. Die Einlassöffnung kann an der Öffnung eines Behälters angeordnet werden. Der Kupplungsabschnitt dient insbesondere dazu, die Inhalationsvorrichtung lösbar an dem Behälter zu befestigen. Durch die Einlassöffnung kann ein Fluid bzw. ein Aerosol oder ein Gas in das Innere der Inhalationsvorrichtung eingebracht und in Richtung des Ansetzabschnitts befördert werden.

Der Ansetzabschnitt weist bevorzugt zumindest eine Ausgabeöffnung auf. Durch die Ausgabeöffnung kann ein Gas und/oder Aerosol abgegeben und insbesondere durch eine die Vorrichtung benutzende Person eingeatmet werden.

Vorzugsweise erstreckt sich ein Luftzufuhrkanal von Luftzufuhröffnungen an der Außenseite der Inhalationsvorrichtung in den Förderkanal. Die Luftzufuhröffnungen sind bevorzugt so angeordnet, dass sie bei Benutzung der Inhalationsvorrichtung nicht abgedeckt bzw. verschlossen werden. Bevorzugt sind die Luftzufuhröffnungen beanstandet von der Ausgabeöffnung oder den Ausgabeöffnungen und von der Einlassöffnung angeordnet. Insbesondere sind die Luftzufuhröffnungen derart ausgebildet, dass sie bei auf einen Behälter aufgesetzter Inhalationsvorrichtung eine Luftzufuhr von außerhalb des Behälters erlauben.

Der Förderkanal oder die Förderkanäle sind gegebenenfalls zum Fördern eines Gases und/oder Aerosols vom Kupplungsabschnitt zum Ansetzabschnitt, insbesondere zur Ausgabeöffnung, eingerichtet. Der Förderkanal oder die Förderkanäle sind also gegebenenfalls zumindest gasdurchlässig. Der wenigstens eine Förderkanal ist insbesondere Teil des Förderabschnitts bzw. er bildet einen wesentlichen Teil des Förderabschnitts.

Die Ausgabeöffnung ist gegebenenfalls zum Inhalieren des Gases und/oder Aerosols durch das Nasenloch, insbesondere durch ein menschliches Nasenloch, eingerichtet.

Die Luftzufuhröffnung ist bevorzugt derart angeordnet, dass sie bei an ein Nasenloch angesetzter Ausgabeöffnung nicht abgedeckt ist.

Die vorliegende Erfindung betrifft gegebenenfalls eine Inhalationsvorrichtung umfassend einen Kupplungsabschnitt, einen Förderabschnitt und einen Ansetzabschnitt. Der Kupplungsabschnitt weist gegebenenfalls eine Einlassöffnung auf und ist zur lösbaren Befestigung der Inhalationsvorrichtung an einer Öffnung eines Behälters eingerichtet. Der Ansetzabschnitt ist gegebenenfalls zum Ansetzen an ein Nasenloch eingerichtet und weist eine Ausgabeöffnung auf. Zwischen der Einlassöffnung und der Ausgabeöffnung verläuft gegebenenfalls ein durchgängiger Förderabschnitt. Zumindest ein Luftzufuhrkanal ist gegebenenfalls vorgesehen, der sich von einer Luftzufuhröffnung an der Außenseite der Inhalationsvorrichtung in den Förderabschnitt erstreckt. Der Förderabschnitt umfasst gegebenenfalls einen Förderkanal, der einen gekrümmten, insbesondere helixförmigen, Verlauf aufweist.

Der gekrümmte Verlauf eines Förderkanals kann dazu beitragen, dass gebildete Flüssigkeitstropfen aus dem einzuatmenden Gas abgeschieden werden, indem sie beispielsweise mit der Wand des Förderkanals kollidieren.

Gegebenenfalls kann vorgesehen sein, dass zumindest zwei Förderkanäle vorgesehen sind, die in Form einer Mehrfachhelix verlaufen. Beispielsweise können 2, 3 oder 4 Förderkanäle vorgesehen sein, die respektive in Form einer Doppelhelix, Tripelhelix oder Vierfachhelix verlaufen.

Gegebenenfalls kann vorgesehen sein, dass die Inhalationsvorrichtung eine Längsausdehnung im Bereich zwischen 5 cm und 15 cm, insbesondere von etwa 6 cm, 7 cm, 8 cm, 9 cm, 10 cm, 11 cm, 12 cm, 13 cm oder 14 cm aufweist. Bevorzugt weisen die Förderkanäle eine Länge von 4 cm bis 10 cm, insbesondere etwa 5 cm, 6 cm, 7 cm, 8 cm oder 9 cm auf.

Gegebenenfalls kann vorgesehen sein, dass zwischen Kupplungsabschnitt und Förderkanal ein Verwirbelungsabschnitt vorgesehen ist. Der Luftzufuhrkanal mündet bevorzugt in den Verwirbelungsabschnitt.

Gegebenenfalls kann vorgesehen sein, dass der Förderkanal bzw. die Förderkanäle ausgehend vom Verwirbelungsabschnitt in Richtung der Ausgabeöffnung verlaufen.

Gegebenenfalls kann vorgesehen sein, dass, bei Vorliegen zumindest zweier Förderkanäle, am Ansetzabschnitt eine Kammer vorgesehen ist, in die die Förderkanäle münden.

Gegebenenfalls kann vorgesehen sein, dass zwei Ansetzabschnitte mit jeweils einer Ausgabeöffnung vorgesehen sind. Die Ansetzabschnitte können zum gleichzeitigen Ansetzen an je ein Nasenloch eingerichtet sein.

Gegebenenfalls kann vorgesehen sein, dass der Luftzufuhrkanal einen ringförmigen Abschnitt aufweist, wobei mehrere Luftzufuhröffnungen in den ringförmigen Abschnitt münden und wobei der ringförmige Abschnitt insbesondere im Verwirbelungsabschnitt angeordnet ist.

Gegebenenfalls kann vorgesehen sein, dass der ringförmige Abschnitt einen Teil des Förderabschnitts umgibt und von diesem Teil des Förderabschnitts durch eine ringförmige Wand abgetrennt ist. Die ringförmige Wand kann insbesondere konzentrisch innerhalb des Verwirbelungsabschnitts angeordnet sein.

Im Kontext dieser Beschreibung kann der Begriff "konzentrisch" eine gleichbleibende Spaltbreite beschreiben. Beispielsweise kann der Verwirbelungsabschnitt ein hohlzylinderförmiger Abschnitt des Kupplungsabschnitts sein. Die ringförmige Wand kann innerhalb des Verwirbelungsabschnitts so angeordnet sein, dass die Achsen des Verwirbelungsabschnitts und der ringförmigen Wand zusammenfallen. Dadurch kann ein ringförmiger Abschnitt des Luftzufuhrkanals mit gleichbleibender Spaltbreite um die ringförmige Wand gebildet sein.

Gegebenenfalls können auch die Achsen der helixförmigen Förderkanäle mit der Achse des Verwirbelungsabschnitts und/oder der ringförmigen Wand und/oder des ringförmigen Abschnitts zusammenfallen.

Gegebenenfalls kann vorgesehen sein, dass im Verwirbelungsabschnitt eine Verwirbelungsvorrichtung vorgesehen ist. Die Verwirbelungsvorrichtung kann Durchlassöffnungen aufweisen, die einen Gasdurchtritt zwischen Kupplungsabschnitt und Förderabschnitt erlauben. Die Durchlassöffnungen sind bevorzugt durch Stege getrennt.

Bei der Beförderung des Gases durch die Durchlassöffnungen können Flüssigkeitstropfen mit den Stegen der Verwirbelungsvorrichtung kollidieren. Dies kann ebenfalls zur Abscheidung von Flüssigkeitstropfen aus dem einzuatmenden Gas beitragen, bevor dieses in ein Nasenloch abgegeben wird.

Gegebenenfalls kann vorgesehen sein, dass die Verwirbelungsvorrichtung zumindest teilweise im Kupplungsabschnitt angeordnet ist.

Gegebenenfalls kann vorgesehen sein, dass die Verwirbelungsvorrichtung einen ersten Abschnitt aufweist, der in Form eines Hohlkegels oder Hohlkegelstumpfs ausgebildet ist, welcher sich in Richtung der Einlassöffnung verjüngt. Insbesondere kann vorgesehen sein, dass die Durchlassöffnungen als Löcher in der Wand des ersten Abschnitts ausgebildet sind.

Gegebenenfalls kann vorgesehen sein, dass die Verwirbelungsvorrichtung einen zweiten Abschnitt aufweist, der in Form eines Zylinders ausgebildet ist.

Gegebenenfalls kann vorgesehen sein, dass der zweite Abschnitt am verjüngten Ende des Hohlkegels oder des Hohlkegelstumpfs des ersten Abschnitts angeordnet ist und dieses Ende gegebenenfalls verschließt.

Gegebenenfalls kann vorgesehen sein, dass am Kupplungsabschnitt, insbesondere an einer Innenfläche des Kupplungsabschnitts, eine Formschlussvorrichtung, bevorzugt ein Gewinde, vorgesehen ist.

Gegebenenfalls kann vorgesehen sein, dass die Inhalationsvorrichtung einstückig ausgebildet ist, und/oder dass die Inhalationsvorrichtung in einem additiven Fertigungsschritt, insbesondere mittels 3D-Druck, hergestellt ist.

Die Herstellung mittels additiver Fertigung ermöglicht die Bereitstellung komplexer innerer Geometrien, die mit anderen Methoden nicht oder nur in Verbindung mehrerer unterschiedlicher Fertigungsschritte bzw. Nachbearbeitungen zugänglich wären.

Die Inhalationsvorrichtung kann einstückig aus Kunststoff gebildet sein, insbesondere aus thermoplastischem Kunststoff.

Die vorliegende Erfindung betrifft gegebenenfalls auch ein System umfassend oder bestehend aus einer Inhalationsvorrichtung und einem Behälter.

Gegebenenfalls kann vorgesehen sein, dass die Inhalationsvorrichtung mit ihrem Kupplungsabschnitt an einer Öffnung des Behälters angeordnet, insbesondere mit dieser Öffnung verbunden, ist. Alternativ oder zusätzlich kann die Verwirbelungsvorrichtung zumindest teilweise in den Behälter ragen.

Gegebenenfalls kann vorgesehen sein, dass der zweite Abschnitt der Verwirbelungsvorrichtung innerhalb des Behälters angeordnet ist.

Beispielsweise kann der zweite Abschnitt im Hals eines als Fläschchen ausgebildeten Behälters angeordnet sein.

Gegebenenfalls kann vorgesehen sein, dass zwischen zweitem Abschnitt und Innenwand des Behälters eine insbesondere umlaufende Freistellung vorgesehen ist. Alternativ oder zusätzlich kann vorgesehen sein, dass der Radius des zweiten Abschnitts bezüglich eines Innendurchmessers des Behälters, insbesondere des Innendurchmessers der Öffnung des Behälters, ein Untermaß aufweist, welches die Größe eines freien Querschnitts zwischen einer Behälterwand und dem zylinderförmigen zweiten Abschnitt vorgibt.

Durch eine derartige Anordnung kann der durch den Luftzufuhrkanal eintretenden Luftstrom umgelenkt und/oder abgebremst werden. Dies kann dazu beitragen, dass die Bildung von Flüssigkeitstropfen reduziert oder verhindert wird.

Gegebenenfalls kann vorgesehen sein, dass ein durchgängiger Strömungspfad von der Luftzufuhröffnung durch zumindest einen Teil des Innenraums des Behälters und zur Ausgabeöffnung verläuft. Alternativ oder zusätzlich kann vorgesehen sein, dass der Innenraum des Behälters gasdurchgängig mit der Ausgabeöffnung verbunden ist.

Gegebenenfalls kann vorgesehen sein, dass der Strömungspfad aufeinanderfolgend durch die Luftzufuhröffnungen, in den Verwirbelungsabschnitt, entlang der Außenseite der ringförmigen Wand und des ersten Abschnitts der Verwirbelungsvorrichtung, durch die Durchlassöffnungen, durch den Förderabschnitt, durch den Ansetzabschnitt und durch die Ausgabeöffnung verläuft.

Insbesondere die Leitung der Strömung entlang der Außenseite der ringförmigen Wand und des ersten Abschnitts der Verwirbelungsvorrichtung kann zur Umlenkung und zum Bremsen der zugeführten Luft beitragen.

Die Inhalationsvorrichtung und/oder das System kann verwendet werden indem die Inhalationsvorrichtung an zumindest ein Nasenloch angesetzt und Gas durch die Ausgabeöffnung eingeatmet wird.

Gegebenenfalls kann bei der Verwendung vorgesehen sein, dass, durch beim Einatmen entstehenden Unterdruck, Luft durch die Luftzufuhröffnungen in die Inhalationsvorrichtung gesaugt wird, sich mit Gas aus dem Behälter vermischt und als Luft-Gas-Gemisch durch den Förderabschnitt zur Ausgabeöffnung transportiert wird.

Gegebenenfalls kann bei der Verwendung vorgesehen sein, dass durch die Luftzufuhröffnungen eintretende Luft entlang der Außenseite der ringförmigen Wand und der Verwirbelungsvorrichtung in den Behälter geleitet wird.

Gegebenenfalls kann bei der Verwendung vorgesehen sein, dass schwere Partikel, insbesondere Flüssigkeitstropfen, im Verlauf des Strömungspfads durch die Inhalationsvorrichtung abgeschieden werden.

Gegebenenfalls kann bei der Verwendung vorgesehen sein, dass die Abscheidung durch Kollision mit und Anhaften an Teilen der Inhalationsvorrichtung erfolgt, wobei Innenwände der Inhalationsvorrichtung, insbesondere die Stege der Verwirbelungsvorrichtung und/oder die gekrümmten Innenwände des Förderabschnitts, Flächen zum Abscheiden bereitstellen.

Die Inhalationsvorrichtung und das System sind unabhängig voneinander. Beliebige Kombinationen von Merkmalen dieser beiden Gegenstände zählen jedoch ebenfalls zum Gegenstand der vorliegenden Offenbarung.

Bestimmte Aspekte der vorliegenden Offenbarung werden nachfolgend anhand von exemplarischen Ausführungsbeispielen im Detail erläutert. Weitere technische Merkmale ergeben sich aus der Beschreibung, den Patentansprüchen sowie den Figuren der Ausführungsbeispiele.

Es zeigen:
Fig. 1 eine aufgeschnittene dreidimensionale Darstellung einer Inhalationsvorrichtung gemäß einem Ausführungsbeispiel;
Fig. 2 eine Schnittdarstellung quer zur Längserstreckung der Inhalationsvorrichtung gemäß dem Ausführungsbeispiel auf Höhe der Luftzufuhröffnungen in einer Ansicht von oben;
Fig. 3 eine Schnittdarstellung entlang der Längserstreckung der Inhalationsvorrichtung in einer Seitenansicht.

Sofern nicht anders angegeben sind in den Figuren die folgenden Merkmale bzw. Elemente dargestellt: Inhalationsvorrichtung 1, Kupplungsabschnitt 2, Förderabschnitt 3, Ansetzabschnitt 4, Einlassöffnung 5, Ausgabeöffnung 6, Luftzufuhrkanal 7, Luftzufuhröffnung 8, Förderkanal 9, Verwirbelungsabschnitt 10, Kammer 11, ringförmiger Abschnitt 12, ringförmige Wand 13, Verwirbelungsvorrichtung 14, Durchlassöffnung 15, Steg 16, erster Abschnitt 17, zweiter Abschnitt 18, Formschlussvorrichtung 19, Strömungspfad 20.

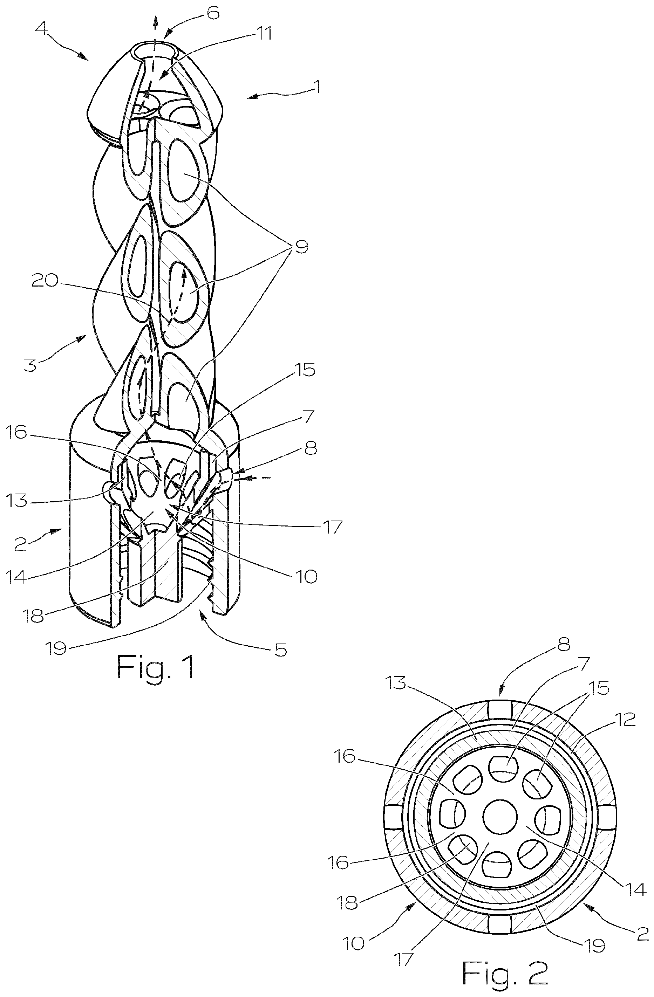

Fig. 1 zeigt eine aufgeschnittene dreidimensionale Darstellung einer Inhalationsvorrichtung 1 gemäß einem Ausführungsbeispiel. Die Inhalationsvorrichtung weist drei Abschnitte auf, nämlich einen Kupplungsabschnitt 2, einen Förderabschnitt 3 und einen Ansetzabschnitt 4.

Der Kupplungsabschnitt 2 ist gemäß dieser Ausführung im Wesentlichen hohlzylinderförmig ausgebildet, wobei anzumerken ist, dass zumindest die äußere Form keinen wesentlichen Einfluss auf die Funktionsweise hat. Die Formschlussvorrichtung 19 zum Anbringen der Inhalationsvorrichtung 1 an einem Behälter (nicht dargestellt) ist in dieser Ausführung als Gewinde, insbesondere als Innengewinde an der Innenseite des hohlzylinderförmigen Kupplungsabschnitts 2 ausgebildet. Im eine formschlüssige Verbindung mit dem Behälter zu erlauben, sollte dieser ein korrespondierendes Außengewinde aufweisen.

Der Kupplungsabschnitt 2, insbesondere die Wand des Hohlzylinders, ist oberhalb der Formschlussvorrichtung 19 von mehreren Luftzufuhröffnungen 8 durchbrochen. Der Kupplungsabschnitt 2 ist über einen ersten Übergang mit dem Förderabschnitt verbunden. Gemäß dieser Ausführung springt der erste Übergang gegenüber der hohlzylindrischen Wand des Kupplungsabschnitts nach innen vor.

Am unteren Ende des Kupplungsabschnitts 2 befindet sich eine Einlassöffnung 5, in die der Behälter teilweise eingesteckt bzw. mit Hilfe der Formschlussvorrichtung 19 eingeschraubt werden kann.

Ein Teil des Kupplungsabschnitts 2 umfasst oder umschließt einen Verwirbelungsabschnitt 10. Der Verwirbelungsabschnitt 10 umfasst eine Verwirbelungsvorrichtung 14. Mit anderen Worten ist der Verwirbelungsabschnitt 10 und/oder dessen Verwirbelungsvorrichtung 14 innerhalb des hohlzylinderförmigen Kupplungsabschnitts 2 angeordnet. Der Verwirbelungsabschnitt 10 bzw. dessen Verwirbelungsvorrichtung 14 können jedoch auch als ein Teil des Kupplungsabschnitts 2 angesehen werden.

Der Verwirbelungsabschnitt umfasst eine ringförmige Wand 13 und die Verwirbelungsvorrichtung 14. Die Verwirbelungsvorrichtung 14 umfasst einen ersten Abschnitt 17 und einen zweiten Abschnitt 18.

Der erste Abschnitt 17 ist gemäß dieser Ausführung im Wesentlichen hohlkegelförmig oder hohlkegelstumpfförmig ausgebildet. Der erste Abschnitt 17 weist durch Stege 16 getrennte Durchlassöffnungen 15 auf. Die Durchlassöffnungen 15 sind in dieser Ausführung als Löcher durch die Wand des hohlkegelförmigen oder hohlkegelstupfförmigen ersten Abschnitts 17 ausgebildet.

Das erweiterte, in dieser Darstellung obere, Ende des ersten Abschnitts geht in die ringförmige Wand 13 über, die seitlich zur Innenseite des hohlzylinderförmigen Kupplungsabschnitt 2 beabstandet ist und somit einen ringförmigen Abschnitt 12 des Luftzufuhrkanals 7 bildet.

Die ringförmige Wand 13 schließt im Bereich zwischen Kupplungsabschnitt 2 und Förderabschnitt 3 nach oben mit dem ersten Übergang, insbesondere gasundurchlässig, ab.

Das verjüngte, in dieser Darstellung untere, Ende des ersten Abschnitts 17 ist mit dem zweiten Abschnitt 18 verbunden bzw. durch diesen verschlossen. Der zweite Abschnitt 18 ist im Wesentlichen zylinderförmig ausgebildet. Beim Einstecken bzw. Einschrauben des Behälters in den Kupplungsabschnitt 2, in die Einlassöffnung 5 und/oder in die Formschlussvorrichtung 19, insbesondere in das Gewinde wird der zweite Abschnitt 18 der Verwirbelungsvorrichtung 14 in den Behälter eingeführt, wobei rings um den zweiten Abschnitt 18 der Verwirbelungsvorrichtung 14 ein gasdurchlässiger Spalt frei bleibt.

In dieser Ausführungsform verläuft der Förderabschnitt 3, insbesondere vom oberen Ende des Kupplungsabschnitts 2 bzw. vom ersten Übergang zum Ansetzabschnitt bzw. zum zweiten Übergang, der den Förderabschnitt 3 mit dem Ansetzabschnitt 4 verbindet. Der Förderabschnitt 3 weist einen oder mehrere Förderkanäle 9 auf. In diesem Ausführungsbeispiel weist der Förderabschnitt 3 drei Förderkanäle 9 auf. Jeder Förderkanal 9 erstreckt sich gekrümmt, insbesondere helixförmig entlang des Förderabschnitts 3 vom Kupplungsabschnitt 2 zum Ansetzabschnitt 4. In diesem Ausführungsbeispiel münden die drei Förderkanäle 9 in einer Kammer 11 des Ansetzabschnitts 4.

Der Ansetzabschnitt 4 ist so geformt, dass er bequem an einem Nasenloch angesetzt oder zumindest teilweise in ein Nasenloch eingeführt werden kann. Der Ansetzabschnitt 4 und insbesondere die am Ansetzabschnitt 4 vorgesehene Ausgabeöffnung 6, sind so ausgebildet, dass das Nasenloch mit dem Ansetzabschnitt 4 dicht abschließen kann, während die Ausgabeöffnung 6 zum Durchlassen von Gas aus dem Ansetzabschnitt 4 der Inhalationsvorrichtung 1 in das Nasenloch frei bleibt. In anderen Worten ist der Ansetzabschnitt 4 so ausgeformt, dass ein Nasenloch mit ihm rings um die Ausgabeöffnung 6 dichtend abschließen kann.

Durch die Luftzufuhröffnungen 8 eintretende Luftströme werden durch die Verwirbelungsvorrichtung 14, insbesondere die ringförmige Wand 13 und/oder den ersten Abschnitt 17 in Richtung Einlassöffnung 5 abgelenkt. Dadurch tritt die zugeführte Luft auch in den Behälter ein. Im Behälter und im Verwirbelungsabschnitt 10 vermischt sich die zugeführte Luft mit dem aus dem Behälter austretenden Gas zu einem Luft-Gas-Strom. Dieser Strom bewegt sich entlang eines Strömungspfads 20 durch die Durchlassöffnungen 15 der Verwirbelungsvorrichtung 14. Dabei werden bereits erste Flüssigkeitstropfen, die mit dem Strom transportiert werden an den Stegen 16 der Verwirbelungsvorrichtung 14 abgeschieden. Nach dem Verwirbelungsabschnitt 10 wird der Strom durch den Förderabschnitt 3 geleitet.

In Fig. 1 ist ein exemplarischer Strömungspfad 20 durch die Inhalationsvorrichtung 1 dargestellt. Es versteht sich, dass viele weitere mögliche, aber nicht dargestellte Strömungspfade 20 durch die Inhalationsvorrichtung 1, und insbesondere durch eine oder mehrere beliebige Luftzufuhröffnungen 8, eine oder mehrere beliebige Durchlassöffnungen15 und/oder eine oder mehrere beliebige Förderkanäle 9, verlaufen.

Im Fall dieses Ausführungsbeispiels wird der Strom am oberen Ende des Kupplungsabschnitts 2 bzw. im Bereich des ersten Übergangs auf die drei Förderkanäle 9 des Förderabschnitts 3 aufgeteilt. Nach Passieren des Förderabschnitts 3 bzw. der Förderkanäle 9 wird der aufgeteilte Strom in der Kammer 11 des Ansetzabschnitts 4, also am oberen Ende des Förderabschnitts 3 bzw. im Bereich des zweiten Übergangs wiedervereinigt und kann die Inhalationsvorrichtung 1 durch die Ausgabeöffnung 6 verlassen.

Fig. 2 zeigt eine Schnittdarstellung quer zur Längserstreckung des Ausführungsbeispiels der Inhalationsvorrichtung 1 auf Höhe der Luftzufuhröffnungen 8.

Zu äußerst ist der hohlzylinderförmige Abschnitt des Kupplungsabschnitts 2 dargestellt. Die hohlzylindrische Wand ist von Luftzufuhröffnungen 8 durchbrochen, die in einem ringförmigen Abschnitt 12 des Luftzufuhrkanals 7 münden. An der Innenseite der hohlzylindrischen Wand des Kupplungsabschnitts 2 ist zudem die Formschlussvorrichtung 19, also das Gewinde zu sehen.

Innerhalb des Kupplungsabschnitts 2 bzw. als Teil des Kupplungsabschnitts 2 ist der Verwirbelungsabschnitt 10 vorgesehen. Der ringförmige Abschnitt 12 verläuft rings um die ringförmige Wand 13 der Verwirbelungsvorrichtung 14 des Verwirbelungsabschnitts 10. Die Darstellung zeigt schraffiert die Schnittflächen durch die hohlzylinderförmige Wand des Kupplungsabschnitts 2 und durch die ringförmige Wand 13 der Verwirbelungsvorrichtung 14. Im Zentrum der Abbildung ist der erste Abschnitt 17 der Verwirbelungsvorrichtung 14 dargestellt. Gemäß dieser Ausführungsform weist der erste Abschnitt 17 acht Durchlassöffnungen 15 auf, die durch Stege 16 voneinander getrennt sind. Durch die Durchlassöffnungen 15 ist zudem der zweite Abschnitt 18 der Verwirbelungsvorrichtung zu erkennen.

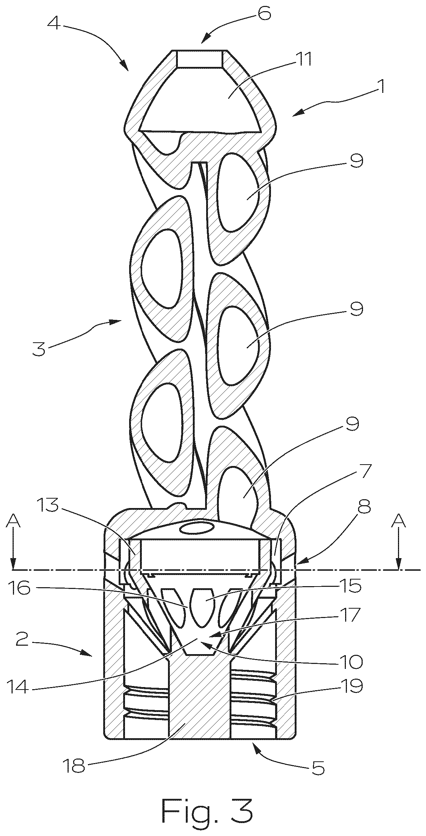

Fig. 3 zeigt eine Schnittdarstellung entlang der Längserstreckung der Inhalationsvorrichtung 1 gemäß einem Ausführungsbeispiel in einer Seitenansicht. Die Inhalationsvorrichtung weist einen Kupplungsabschnitt 2, einen Förderabschnitt 3 und einen Ansetzabschnitt 4 auf. Am Kupplungsabschnitt 2 ist eine Einlassöffnung 5 vorgesehen.

Am Ansetzabschnitt 4, gemäß der Fig. 3 am oberen Ende der Inhalationsvorrichtung 1, ist eine Ausgabeöffnung 6 vorgesehen. Die Inhalationsvorrichtung 1 weist in ihrem Kupplungsabschnitt einen Luftzufuhrkanal 7 auf. Der Luftzufuhrkanal 7 ist über Luftzufuhröffnungen 8 luftdurchlässig mit der Außenseite der Inhalationsvorrichtung 1, genauer gesagt mit der Außenseite des Kupplungsabschnitts 2 verbunden. Der Förderabschnitt 3 weist in dem Ausführungsbeispiel gemäß Fig. 3 drei Förderkanäle 9 auf. Die Förderkanäle 9 sind in Form einer Dreifachhelix angeordnet.

Die Inhalationsvorrichtung 1 ist zwischen der Einlassöffnung 5 und der Ausgabeöffnung 6 durchgängig für Gas, insbesondere Luft und/oder gasförmigen Wirkstoff, ausgebildet. Ebenso ist die Inhalationsvorrichtung 1 zwischen den Luftzufuhröffnungen 8 und der Ausgabeöffnung 6 durchgängig für Gas, insbesondere Luft und/oder gasförmigen Wirkstoff, ausgebildet. Im Verlauf dieses Durchgangs ist zwischen der Einlassöffnung 5 und den Förderkanälen 9 bzw. zwischen dem Luftzufuhrkanal 7 und den Förderkanälen 9 ein Verwirbelungsabschnitt 10 vorgesehen.

Luft kann durch die Luftzufuhröffnungen 8 und den Luftzufuhrkanal 7 in die Inhalationsvorrichtung 1 eintreten. Im Bereich des Verwirbelungsabschnitts 10 vermischt sich die zugeführte Luft mit gasförmigem Wirkstoff, der durch die Einlassöffnung 5 in die Inhalationsvorrichtung 1 eintritt. Es bildet sich ein Luft-WirkstoffGemisch das anschließend an den Verwirbelungsabschnitt 10 durch die Förderkanäle 9 und die Kammer 11 zur Ausgabeöffnung 6 befördert werden kann.

Der Verwirbelungsabschnitt 10 ist in dieser Ausführungsform innerhalb des Kupplungsabschnitts 2 vorgesehen. Im Verwirbelungsabschnitt 10 ist eine ringförmige Wand 13 angeordnet, die die Wand des Kupplungsabschnitts 2 mit einer Verwirbelungsvorrichtung 14 verbindet. Die ringförmige Wand 13 ist gasundurchlässig. Zwischen der ringförmigen Wand 13 des Verwirbelungsabschnitts 10 und der Wand des Kupplungsabschnitts 2 liegt der Luftzufuhrkanal 7. Die ringförmige Wand 13 lenkt eintretende Luft in Richtung der Einlassöffnung 5 um die Durchmischung der Luft mit dem Wirkstoff zu verbessern.

Diese Durchmischung wird weiter vorangetrieben durch den ersten Abschnitt 17 der Verwirbelungsvorrichtung 14 der hohlkegelstumpfförmig mit dem zulaufenden Ende in Richtung der Einlassöffnung 5 im Verwirbelungsabschnitt 10 angeordnet ist. Die Verwirbelungsvorrichtung 14 weist im ersten Abschnitt 17 gasdurchlässige Durchlassöffnungen 15 auf, die durch Stege 16 getrennt sind. Im Gegensatz zur ringförmigen Wand 13 kann ein Gas dadurch durch die Verwirbelungsvorrichtung 14 hindurchtreten.

Die Verwirbelungsvorrichtung 14 weist am zulaufenden Ende des hohlkegelstumpfförmigen ersten Abschnitts 17 einen zylindrischen zweiten Abschnitt 18 auf der bei bestimmungsgemäßer Anordnung der Inhalationsvorrichtung 1 an einem Behälter, wie zum Beispiel an einem Fläschchen in den Hals dieses Fläschchens ragen soll ohne diesen jedoch vollständig zu blockieren. Zur Befestigung der Inhalationsvorrichtung 1 an einem solchen Behälter ist eine Formschlussvorrichtung 19 vorgesehen, die im Ausführungsbeispiel gemäß Fig. 3 als Gewinde an der Innenseite des Kupplungsabschnitts 2 beziehungsweise der Einlassöffnung 5 vorgesehen ist.

Darüber hinaus ist in Fig. 3 eine Ebene A-A dargestellt die sich auf Höhe der Luftzufuhröffnungen 8 befindet. Der Schnitt gemäß Fig. 2 ist entlang dieser Ebene A-A durchgeführt, wobei anzumerken ist, dass Fig. 2 und 3 nicht notwenderweise dasselbe Ausführungsbeispiel zeigen und die Ebene A-A lediglich zur exemplarischen Visualisierung gezeigt ist.

In nicht dargestellten Ausführungsformen können auch andere Anzahlen von Durchlassöffnungen 15, beispielsweise vier, fünf, sechs, sieben, neun oder zehn Durchlassöffnungen 15 vorgesehen sein.

In einem ebenfalls nicht dargestellten Ausführungsbeispiel kann der Ansetzabschnitt 4 zwei Ausgabeöffnungen 6 umfassen, die zum gleichzeitigen Ansetzen an jeweils ein Nasenloch eingerichtet sind. Insbesondere ist in diesem Ausführungsbeispiel vorgesehen, dass der Gasstrom in einer im Ansetzabschnitt 4 vorgesehenen Kammer 11 aufgeteilt wird.

## Patentansprüche

1. **Inhalationsvorrichtung** (1) umfassend einen Kupplungsabschnitt (2), einen Förderabschnitt (3) und einen Ansetzabschnitt (4),
- wobei der Kupplungsabschnitt (2) eine Einlassöffnung (5) aufweist und zur lösbaren Befestigung der Inhalationsvorrichtung (1) an einer Öffnung eines Behälters eingerichtet ist,
- wobei der Ansetzabschnitt (4) zum Ansetzen an ein Nasenloch eingerichtet ist und eine Ausgabeöffnung (6) aufweist,
- wobei zwischen Einlassöffnung (5) und Ausgabeöffnung (6) ein durchgängiger Förderabschnitt (3) verläuft, und
- wobei zumindest ein Luftzufuhrkanal (7) vorgesehen ist, der sich von einer Luftzufuhröffnung (8) an der Außenseite der Inhalationsvorrichtung in den Förderabschnitt (3) erstreckt,
- wobei der Förderabschnitt (3) zumindest einen Förderkanal (9) umfasst, der einen gekrümmten, insbesondere helixförmigen, Verlauf aufweist
- wobei zwischen Kupplungsabschnitt (2) und Förderkanal (9) ein Verwirbelungsabschnitt (10) vorgesehen ist, wobei der Luftzufuhrkanal (8) in den Verwirbelungsabschnitt (10) mündet,
**dadurch gekennzeichnet, dass** im Verwirbelungsabschnitt (10) eine Verwirbelungsvorrichtung (14) vorgesehen ist, wobei die Verwirbelungsvorrichtung (14) Durchlassöffnungen (15) aufweist, die einen Gasdurchtritt zwischen Kupplungsabschnitt (2) und Förderabschnitt (3) erlauben.

2. Inhalationsvorrichtung (1) nach Anspruch 1, **dadurch gekennzeichnet, dass** zumindest zwei Förderkanäle (9) vorgesehen sind, die in Form einer Mehrfachhelix verlaufen.

3. Inhalationsvorrichtung (1) nach Anspruch 1 oder 2, **dadurch gekennzeichnet, dass** der Förderkanal (9) bzw. die Förderkanäle (9) ausgehend vom Verwirbelungsabschnitt (10) in Richtung der Ausgabeöffnung (6) verlaufen.

4. Inhalationsvorrichtung (1) nach einem der Ansprüche 1 bis 3, **dadurch gekennzeichnet, dass** zumindest zwei Förderkanäle (9) vorgesehen sind und am Ansetzabschnitt (4) eine Kammer (11) vorgesehen ist, in die die Förderkanäle (9) münden.

5. Inhalationsvorrichtung (1) nach einem der Ansprüche 1 bis 4, **dadurch gekennzeichnet, dass** zwei Ansetzabschnitte (4) mit jeweils einer Ausgabeöffnung (6) vorgesehen sind, wobei die Ansetzabschnitte (4) zum gleichzeitigen Ansetzen an je ein Nasenloch eingerichtet sind.

6. Inhalationsvorrichtung (1) nach einem der Ansprüche 1 bis 5, **dadurch gekennzeichnet,**
- **dass** der Luftzufuhrkanal (7) einen ringförmigen Abschnitt (12) aufweist, wobei mehrere Luftzufuhröffnungen (8) in den ringförmigen Abschnitt (12) münden, und wobei der ringförmige Abschnitt (12) insbesondere im Verwirbelungsabschnitt (10) angeordnet ist
- und vorzugsweise dass der ringförmige Abschnitt (12) einen Teil des Förderabschnitts (3) umgibt und von diesem Teil des Förderabschnitts (3) durch eine ringförmige Wand (13) abgetrennt ist, wobei die ringförmige Wand (13) insbesondere konzentrisch innerhalb des Verwirbelungsabschnitts (10) angeordnet ist.

7. Inhalationsvorrichtung (1) nach einem der Ansprüche 1 bis 6, **dadurch gekennzeichnet, dass** die Durchlassöffnungen (15) durch Stege (16) getrennt sind.

8. Inhalationsvorrichtung (1) nach Anspruch 1 bis 7, **dadurch gekennzeichnet, dass** die Verwirbelungsvorrichtung (14) zumindest teilweise im Kupplungsabschnitt (2) angeordnet ist.

9. Inhalationsvorrichtung (1) nach einem der Ansprüche 1 bis 8, **dadurch gekennzeichnet,**
- **dass** die Verwirbelungsvorrichtung (14) einen ersten Abschnitt (17) aufweist, der in Form eines Hohlkegels oder Hohlkegelstumpfs ausgebildet ist, welcher sich in Richtung der Einlassöffnung (5) verjüngt, wobei insbesondere die Durchlassöffnungen (15) als Löcher in der Wand des ersten Abschnitts (17) ausgebildet sind
- und/oder dass die Verwirbelungsvorrichtung (14) einen zweiten Abschnitt (18) aufweist, der in Form eines Zylinders ausgebildet ist,
wobei vorzugsweise vorgesehen ist, dass der zweite Abschnitt (18) am verjüngten Ende des Hohlkegels oder des Hohlkegelstumpfs des ersten Abschnitts (17) angeordnet ist und dieses Ende gegebenenfalls verschließt.

10. Inhalationsvorrichtung (1) nach einem der Ansprüche 1 bis 9, **dadurch gekennzeichnet, dass** am Kupplungsabschnitt (2), insbesondere an einer Innenfläche des Kupplungsabschnitts (2), eine Formschlussvorrichtung (19), insbesondere ein Gewinde, vorgesehen ist.

11. Inhalationsvorrichtung (1) nach einem der Ansprüche 1 bis 10, **dadurch gekennzeichnet, dass** die Inhalationsvorrichtung (1) einstückig ausgebildet ist, und/oder dass die Inhalationsvorrichtung (1) in einem additiven Fertigungsverfahren, insbesondere mittels 3D-Druck, hergestellt ist.

12. **System** umfassend oder bestehend aus einer Inhalationsvorrichtung (1) nach einem der Ansprüche 1 bis 11 und einem Behälter.

13. System nach Anspruch 12, **dadurch gekennzeichnet, dass** die Inhalationsvorrichtung (1) mit ihrem Kupplungsabschnitt (2) an einer Öffnung des Behälters angeordnet, insbesondere mit dieser Öffnung verbunden, ist, und/oder dass die Verwirbelungsvorrichtung (14) zumindest teilweise in den Behälter ragt.

14. System nach einem der Ansprüche 12 oder 13, **dadurch gekennzeichnet,**
- **dass** der zweite Abschnitt (18) der Verwirbelungsvorrichtung (14) innerhalb des Behälters angeordnet ist
- und vorzugsweise dass zwischen zweitem Abschnitt (18) und Innenwand des Behälters eine insbesondere umlaufende Freistellung vorgesehen ist, und/oder dass der Radius des zweiten Abschnitts (18) bezüglich eines Innendurchmessers des Behälters, insbesondere des Innendurchmessers der Öffnung des Behälters, ein Untermaß aufweist, welches die Größe eines freien Querschnitts zwischen einer Behälterwand und dem zylinderförmigen zweiten Abschnitt (18) vorgibt.

15. System nach einem der Ansprüche 12 bis 14, **dadurch gekennzeichnet,**
- **dass** ein durchgängiger Strömungspfad (20) von der Luftzufuhröffnung (8) durch zumindest einen Teil des Innenraums des Behälters und zur Ausgabeöffnung (6) verläuft,
- und/oder dass der Innenraum des Behälters gasdurchgängig mit der Ausgabeöffnung (6) verbunden ist
wobei vorzugsweise vorgesehen ist, dass der Strömungspfad (20) aufeinanderfolgend durch die Luftzufuhröffnungen (8), in den Verwirbelungsabschnitt (10), entlang der Außenseite der ringförmigen Wand (13) und des ersten Abschnitts (17) der Verwirbelungsvorrichtung (14), durch die Durchlassöffnungen (15), durch den Förderabschnitt (3), durch den Ansetzabschnitt (4) und durch die Ausgabeöffnung (6) verläuft.
